# EUROPEAN PATENT SPECIFICATION

(11) **EP 2 377 949 B1**
(45) Date of publication and mention of the grant of the patent: **21.11.2018**
(21) Application number: 09831393.5
(22) Date of filing: 11.12.2009
(51) Int. Cl.: G06F 19/22, C12Q 1/68

(54) **CONSTRUCTION METHOD AND SYSTEM OF FRAGMENTS ASSEMBLING SCAFFOLD**
KONSTRUKTIONSVERFAHREN UND SYSTEM FÜR FRAGMENTASSEMBLIERUNGSGERÜST
PROCÉDÉ DE CONSTRUCTION ET SYSTÈME D ÉCHAFAUDAGE D'ASSEMBLAGE DE FRAGMENTS

(30) Priority: 12.12.2008 CN 200810218342
(43) Date of publication of application: 19.10.2011
(73) Proprietor: BGI Tech Solutions Co., Ltd., Shenzhen, Guangdong 518083 (CN)
(72) Inventor: NI, Peixiang, Shenzhen Guangdong 518083 (CN); FANG, Xiaodong, Shenzhen Guangdong 518083 (CN); WANG, Jun, Shenzhen Guangdong 518083 (CN); YANG, Huanming, Shenzhen Guangdong 518083 (CN); WANG, Jian, Shenzhen Guangdong 518083 (CN)
(74) Representative: Held, Stephan
(86) International application number: PCT/CN2009/001428
(87) International publication number: WO 2010/066116

(56) References cited:
- WO-A2-01/63543
- WO-A2-01/63543
- CN-A- 1 360 057
- CN-A- 101 504 697
- Mihai Pop ET AL: "Hierarchical scaffolding with Bambus", Genome research, 1 January 2004 (2004-01-01), pages 149-159, XP055150814, United States DOI: 10.1101/gr.1536204 Retrieved from the Internet: URL:http://www.ncbi.nlm.nih.gov/pubmed/147 07177
- Pan-Gyu Kim ET AL: "A Scaffold Analysis Tool Using Mate-Pair Information in Genome Sequencing", Journal of biomedicine & biotechnology, 1 January 2008 (2008-01-01), pages 675741-8, XP055150824, United States DOI: 10.1155/2008/675741 Retrieved from the Internet: URL:http://dx.doi.org/10.1155/2008/675741
- J. WANG: "RePS: A Sequence Assembler That Masks Exact Repeats Identified from the Shotgun Data", GENOME RESEARCH, vol. 12, no. 5, 1 May 2002 (2002-05-01), pages 824-831, XP055150832, DOI: 10.1101/gr.165102
- HAN YUJUN ET AL.: 'Applications of the double-barreled data in whole-genome shotgun sequence assembly and analysis' SCIENCE IN CHINA SER. C LIFE SCIENCES vol. 48, no. 3, 2005, pages 300 - 306, XP008143825
- PEVZNER PAVEL A. ET AL: 'Fragment assembly with double-barreled data' BIOINFORMATICS vol. 17, no. 1, 2001, pages S225 - S233, XP008140748

## Description

### Field of the Invention

The present invention relates to gene engineering filed, in particular a construction method and system of fragments assembling scaffold and a genome sequencing device.

### Background of Invention

Genomics is to study and analyze the whole genetic information of an organism, in order to know the mechanism and function of the whole genetic information. One basic step in genomics is to obtain the whole sequences of on organism. Currently, there is the First-Generation Sequencing Method such as whole genome shotgun sequencing (Sanger Method), as well as the Second-Generation Sequencing Method such as Solexa, Solid and 454 method.

The Sanger Method is briefly described as follows: the whole genome is broken up into small DNA fragments of varying length to construct the Shotgun library; the Shotgun library is randomly sequenced; the sequences fragments are then assembled into whole genome sequence by bioinformatics method. This method is characterized by long sequencing reads.

The Solexa Method is briefly described as follows: the whole genome is broken up into DNA fragments of 100-200bp. Then an adaptor is linked to the DNA fragments and a library is obtained by polymerase chain reaction. The adaptor linked DNA fragment is subsequently immobilized to adaptor linked flow cell. After reaction, different DNA fragments are amplified. In the next step, a sequencing-by-synthesis step is performed using 4 fluorescence labeled dyes. This method is characterized by high throughput, low cost, low sequencing error and short sequencing reads.

The construction of fragments assembling scaffold has always been the key step in *de novo* assembling, which is used for determining the position relation between contigs and building the basic framework for genome assembling. The quality of this process directly affects the final result of the whole genome sequences. The current construction method of scaffold is to link those sequencing fragments that have overlaps, so as to finish the task of assembling sequencing fragments. In the case of short sequencing reads, the overlaps between sequencing fragments is relatively short; thus leading to a low precision for current construction method of scaffold. Given that the Second-Generation Sequencing Method such as Solexa, Solid and 454 method has a shorter sequencing reads than the First-Generation Sequencing Method, current construction method of scaffold can hardly apply to the Second-Generation Sequencing Method to finish the task of assembling sequencing fragments.

Pop et al, Genome Research, 2004, pp. 149-159 discloses a method for constructing a scaffold by taking into account the gap sizes between contigs and untangling ambiguous scaffolds into a collection of linear scaffolds.

WO 01/63543 discloses a method and a system for generating a consensus nucleotide sequence from shot-gun data by taking gap sizes into account and by masking repeat contigs.

### Summary of the Invention

A first object of the present invention is to provide a construction method of fragments assembling scaffold to solve the above mentioned problem.

In one aspect, the present invention provides a computer-implemented method of constructing a scaffold, the method comprising the steps of:
mapping multiple pairs of double-barreled data obtained through sequencing to contigs;
obtaining gap size between said contigs by calculating mean or median length between contigs based on the multiple pairs of double-barreled data mapped to contigs;
constructing a scaffold based on the gap size between contigs and the relative orientation of contigs;
detecting repeat contigs in said scaffold, and masking the detected repeat contigs, wherein a repeat contig is a contig that is linked in one direction to a plurality of contigs that have overlaps;
linearizing the scaffold based on the gap size between contigs and the relative orientation of contigs in the scaffold; and
recalculating the gap size between contigs in the linearized scaffold, wherein the recalculation step comprises: based on the position relationship of the contigs in the linearized scaffold, recalculating the gap size between each two adjacent contigs; and relinking adjacent contigs.

A second object of the present invention is to provide a system for constructing a scaffold, the system comprising means for carrying out the method of claim 1.

In the embodiments of the present invention, by mapping the double-barreled data obtained through sequencing to contigs and obtaining the gap size between said contigs based on multiple pairs of double-barreled data between said contigs, the estimation precision of gap size between contigs in the construction of fragments assembling scaffold is improved greatly. Then the fragments assembling scaffold is constructed based on the gap size between contigs and the double-barreled relation between contigs, and a complete fragments assembling scaffold graph is obtained. As such, even if a genome sequencing method with short sequencing reads is used, it is also possible to finish the task of assembling sequencing fragments. Meanwhile, the error rate in assembling sequencing fragments is reduced.

### Brief Description of the Drawings

Fig. 1 is a flow chart of one example of the construction method of fragments assembling scaffold of the present invention.
Fig. 2 is a flow chart of another example of the construction method of fragments assembling scaffold of the present invention.
Fig. 3 is a representative diagram of a scaffold graph constructed by mapping the double-barreled data to contigs.
Fig. 4 is a diagram showing the masking of repeat contigs.
Figs. 5a and 5b is a representative diagram of a linearized scaffold graph.
Fig. 6 is a diagram showing the recover of repeat contigs.
Fig. 7 is a diagram of one example of the construction system of fragments assembling scaffold of the present invention.
Fig. 8 is a diagram of another example of the construction system of fragments assembling scaffold of the present invention.

### Detailed Description of the Invention

To make the objects, technical solutions and advantages of the present invention more clear and readily to understand, the present invention is further described below in details by referring to accompanying Figures and examples. It should be understood that the described embodiments are to illustrate the present invention only, and not intend to be limiting. In the Figures, identical reference indicates the same or similar component or element.

In the embodiments of the present invention, by mapping the double-barreled data obtained through sequencing to contigs and calculating the gap size between said contigs based on multiple pairs of double-barreled data, the fragments assembling scaffold is constructed based on the gap size between contigs and the double-barreled relation between contigs, and a complete fragments assembling scaffold graph is obtained.

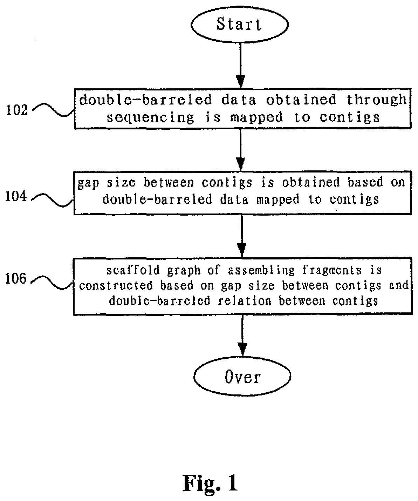

Fig. 1 shows the flow chart of one example of the construction method of fragments assembling scaffold of the present invention.

Referring to Fig. 1, in step 102, double-barreled data (also referred to as double-barreled reads) obtained through sequencing is mapped to contigs.

In the examples of the present invention, the genome may be sequenced through various sequencing methods, such as the First Generation Sequencing Method, the Second Generation Sequencing Method, thereby obtaining multiple short sequences having double-barreled relationships (designated as double-barreled data). In one example of the present invention, the genome is sequenced through the Second Generation Sequencing Method, which method is characterized by high throughout and short sequencing reads, thereby reducing the complexity of the construction method of scaffold.

Various mapping methods may be used to map double-barreled data obtained through sequencing to contigs, such as soap, eland, maq or BLAT mapping program. Upon mapping the double-barreled data obtained through sequencing to contigs, the positions and orientations of double-barreled data on contigs would be obtained.

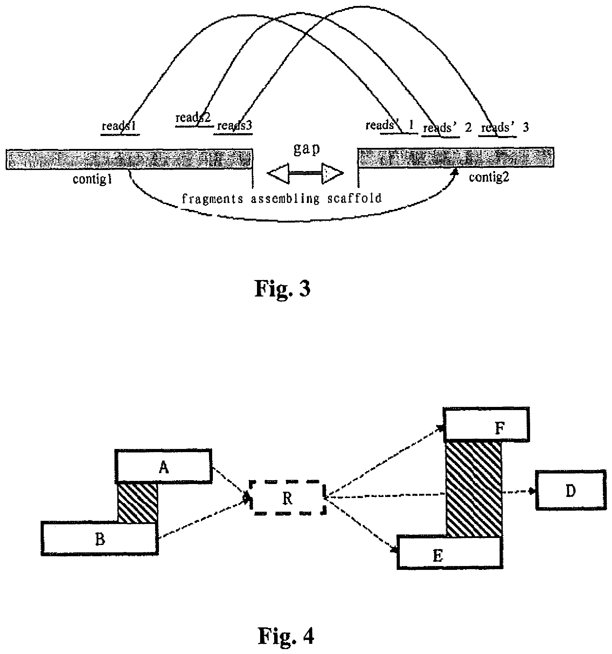

For a case where double-barreled data obtained through sequencing is reads 1 and reads1', reads2 and reads2', reads3 and reads3', Fig. 3 shows a representative scaffold graph after mapping the double-barreled data to contigs.

In step 104, the gap size between the contigs is obtained based on the double-barreled data mapped to the contigs.

Two contigs are linked together via one pair of double-barreled data. The gap length between two contigs can be calculated based on each pair of double-barreled data mapped to the contigs. If there are multiple pairs of double-barreled data between two contigs, then calculate each gap length therefrom and the median or mean gap length is taken as the final gap size between two contigs.

In one example of the present invention, the number of the double-barreled data over two contigs are recorded and marked as weight. A particular threshold is chosen as appropriate. Those cases where the weight is higher than the threshold are considered as effective linking, in order to increase the accuracy of the linking relationship.

In the examples of the present invention, the respective gap size between contigs are calculated based on multiple pairs of double-barreled data between two contigs, the mean gap size is taken as the gap size between those contigs. Referring to Fig. 3, when there are 3 pairs of double-barreled data between contig 1 and contig 2, then 3 gap sizes are calculated based on these 3 pairs of double-barreled data. The mean of these 3 gap sizes is taken as the gap size between contig 1 and contig 2. When determining the gap size between contigs, the gap size between all contigs having double-barreled relationships are calculated, the mean gap size is taken as the gap size between these contigs. Meanwhile, the number of the double-barreled data between contigs is marked as weight (the number of the double-barreled data between contigl and contig2 is 3). When the weight is higher than the predefined threshold, the link between contigl and contig2 is considered as effective link, in order to increase the accuracy of the linking relationship.

If the gap size between two contigs calculated based on one pair of double-barreled data is Xi, which follows a normal distribution of N (µ, σ^2), in which µ denotes the expected value while σ^2 denotes the variance, then the mean gap size between contigs calculated from N pairs of double-barreled data follows the distribution of N (µ, σ^2/N). As such, when the covering degree of double-barreled data on the contigs is high, the estimation precision of gap size between contigs would be improved greatly.

In step 106, the scaffold between contigs is constructed based on the gap size between contigs and the double-barreled relationship between contigs, and a complete scaffold graph is constructed based on each contig, wherein the double-barreled relationship between contigs may be directly determined by the position relationship given by raw experimental data.

Referring to Fig. 3, the gap size between contigl and contig2 is calculated from 3 pairs of double-barreled data between contigl and contig2 as shown in Fig.3, then the scaffold between contigl and contig2 may be constructed based on the gap size between contigl and contig2 and the double-barreled relationship between contigl and contig2, as shown in Fig. 3. Similarly, the scaffold of all contigs having double-barreled relationship may be constructed based on the gap size of all contigs having double-barreled relationship and the double-barreled relationship of all contigs having double-barreled relationship, thereby linking all contigs having double-barreled relationship to obtain the complete scaffold graph, as shown in Fig. 4.

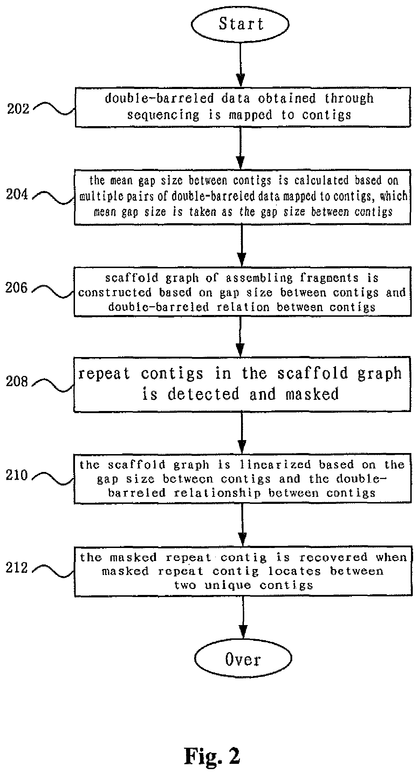

Fig. 2 shows the flow chart of another example of the construction method of fragments assembling scaffold of the present invention.

As shown in Fig. 2, in step 202, double-barreled data obtained through sequencing is mapped to contigs.

In step 204, the mean gap length between the contigs is calculated based on multiple pairs of double-barreled data mapped to the contigs, which is taken as the gap size between the contigs.

In step 206, a scaffold graph is constructed based on the gap size between contigs and the double-barreled relationship between contigs.

In step 208, the constructed scaffold graph is checked for repeat contigs. The detected repeat contigs are masked. It is possible that there is a plurality of repeat contigs in the scaffold graph constructed according to the above discussed method, thereby reducing the accuracy of genome sequencing. By masking repeat contigs in this step, the accuracy of genome sequencing would be increased.

In the examples of the present invention, if one contig is linked in one direction to a plurality of contigs having overlaps, then this contig is considered as repeat contig. Repeat contigs are masked upon detection.

For a scaffold constructed as shown in Fig. 4, since contig R is linked to contig A and contig B in the reverse direction, and there is overlap between contig A and contig B; meanwhile contig R is linked to contig D, contig E and contig F in the forward direction, and there is overlap between contig E and contig F, thereby contig R is a repeat contig, which would be masked.

In order to obtain scaffold of sufficient length within a controllable error range and allow to determine the proper position relationship of as many contigs as possible, in another example of the present invention, the construction method of scaffold further comprises the steps of:
in step 210, the scaffold graph is linearized based on the gap size between contigs and the double-barreled relationship between contigs.

In the examples of the present invention, when repeat contigs are contained in the scaffold constructed in step 206, such repeat contigs are masked via step 208, after masking, the scaffold graph is linearized. When no repeat contig is contained in the scaffold constructed in step 206, the scaffold graph would be linearized directly. The step of linearization is as follows:
placing each contig at appropriate position in the sub-graph based on the gap size between contigs and the double-barreled relationship between contigs, if no significant overlap exists between any two contigs, then performing linearization according to the position relationship of these two contigs.

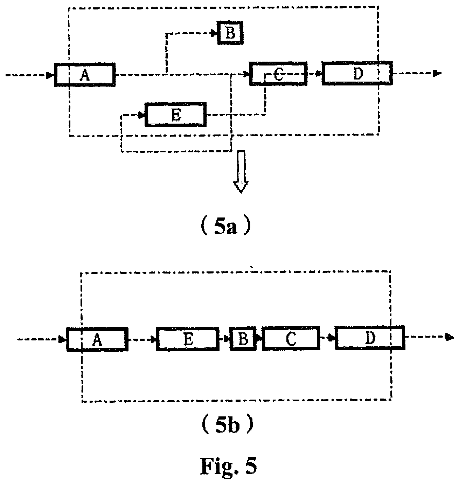

For a scaffold as shown in Fig. 5a, wherein the gap size and the double-barreled relationship between contig A and contig B, the gap size and the double-barreled relationship between contig E and contig D, the gap size and the double-barreled relationship between contig A and contig E, the gap size and the double-barreled relationship between contig E and contig C are known, the linear structural relationship would be deduced therefrom as AEBCD. In other words, the scaffold graph as shown in Fig. 5a can be linearized as the scaffold graph as shown in Fig. 5b directly.

The gap size between contigs in the scaffold might be changed due to the linearization of the scaffold graph. In order to present the gap size between contigs in the linearized scaffold graph accurately, the construction method of scaffold of the present invention further comprises:
recalculating the gap size between contigs in the linearized scaffold graph.

The step of recalculating the gap size between contigs in the linearized scaffold graph comprises: based on the position relationship of the contigs in the linearized scaffold graph, recalculating the gap size between each two adjacent contigs; and relinking adjacent contigs, thereby converting the scaffold graph into a true linear structure. Referring to Figs. 5a and 5b, after converting the linking relationship of AB, AC, EC, ED of Fig. 5a into the linking relationship of AE, EB, BC, CD of Fig. 5b, the gap size of each contigs is calculated from the gap size that has already been obtained. For example, the gap size of AE can be calculated simply as AE=AC-EC.

After performing masking of repeat contigs in the scaffold graph and linearization of sub-graph, it is possible that previously masked repeat contig locates between two unique contigs, since the gap size between contigs in the scaffold have been changed. In this case, in order to reduce the internal gap size in the scaffold and allow the scaffold to be filled as much as possible, the construction method of scaffold further comprises the steps of:
in step 212, when masked repeat contig locates between two unique contigs, recovering the masked repeat contig.

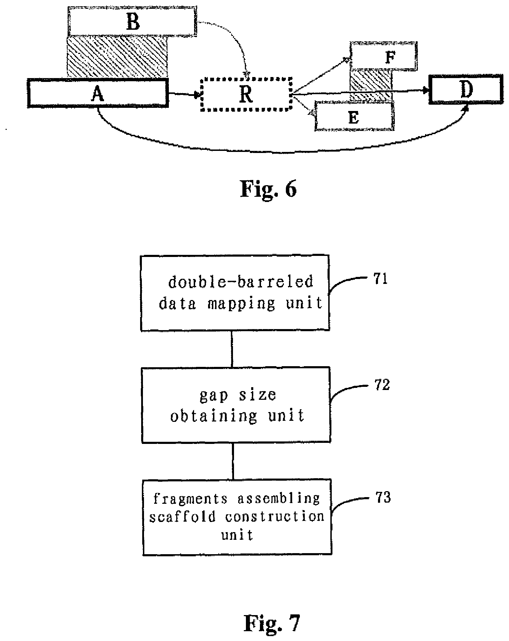

Referring to Fig. 6, which shows the scaffold graph obtained after step 208 and step 210. If the previously masked contig R locates between unique contig A and unique contig D of the scaffold graph, the previously masked contig R would be recovered directly.

Fig. 7 shows a diagram of one example of the construction system of fragments assembling scaffold of the present invention. As shown in Fig. 7, the construction system of fragments assembling scaffold comprises a double-barreled data mapping unit 71; a gap size obtaining unit 72; and a scaffold (fragments assembling scaffold) construction unit 73, wherein:
the double-barreled data mapping unit 71 is used for mapping the double-barreled data obtained through sequencing to contigs. In the examples of the present invention, the genome may be sequenced through various sequencing methods, such as the First Generation Sequencing Method, the Second Generation Sequencing Method, thereby obtaining multiple short sequences having double-barreled relationships (designated as double-barreled data). In one example of the present invention, the genome is sequenced through the Second Generation Sequencing Method, which method is characterized by high throughout and short sequencing reads, thereby reducing the complexity of the construction method of scaffold. Various mapping methods may be used to map double-barreled data obtained through sequencing to contigs, such as soap, eland, maq or BLAT mapping program. Upon mapping the double-barreled data obtained through sequencing to contigs, the positions and orientations of double-barreled data on contigs would be obtained. Fig. 3 shows a representative scaffold graph after mapping the double-barreled data to contigs.

The gap size obtaining unit 72 is used for obtaining the gap size between the contigs based on the double-barreled data mapped to the contigs. For example, the mean or median gap length calculated from multiple pairs of double-barreled data mapped to the contigs is taken as the gap size between two contigs. In addition, the number of the double-barreled data over two contigs are recorded and marked as weight.

In the examples of the present invention, if the gap size between two contigs calculated based on one pair of double-barreled data is Xi, which follows a normal distribution of N (µ, σ^2), in which µ denotes the expected value while σ^2 denotes the variance, then the mean gap size between contigs calculated from N pairs of double-barreled data follows the distribution of N (µ, σ^2/N). As such, when the covering degree of double-barreled data on the contigs is high, the estimation precision of gap size between contigs would be improved greatly.

The scaffold construction unit 73 is used for constructing fragments assembling scaffold based on the gap size between contigs and the double-barreled relation between contigs, and obtaining fragments assembling scaffold graph, wherein the double-barreled relationship between contigs may be directly determined by the position relationship given by raw experimental data.

Referring to Fig. 3, the gap size between contigl and contig2 is calculated from 3 pairs of double-barreled data between contigl and contig2 as shown in Fig.3, then the scaffold between contigl and contig2 may be constructed based on the gap size between contigl and contig2 and the double-barreled relationship between contigl and contig2, as shown in Fig. 3. Similarly, the scaffold of all contigs having double-barreled relationship may be constructed based on the gap size of all contigs having double-barreled relationship and the double-barreled relationship of all contigs having double-barreled relationship, thereby linking all contigs having double-barreled relationship to obtain the complete scaffold graph, as shown in Fig. 4.

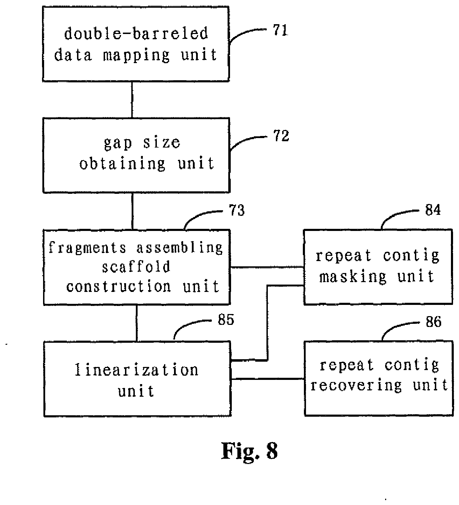

Fig. 8 shows a diagram of another example of the construction system of fragments assembling scaffold of the present invention. As shown in Fig. 8, the construction system of fragments assembling scaffold comprises a double-barreled data mapping unit 71; a gap size obtaining unit 72; and a scaffold construction unit 73; and optionally, a repeat contig masking unit 84; a linearization unit 85 and a repeat contig recovering unit 86, wherein the double-barreled data mapping unit 71, the gap size obtaining unit 72 and the scaffold construction unit 73 is the same as that in Fig. 7. Please refer to the description above.

It is possible that there is a plurality of repeat contigs in the scaffold graph constructed by the scaffold construction unit 73, thereby reducing the accuracy of genome sequencing. In order to increase the accuracy of genome sequencing, in another example of the present invention, the construction system of scaffold further comprises a repeat contig masking unit 84. The repeat contig masking unit 84 detects and masks repeat contigs in the scaffold graph. In the examples of the present invention, if one contig is linked in one direction to a plurality of contigs that having overlaps, then this contig is considered as a repeat contig.

In order to obtain scaffold of sufficient length within a controllable error range and allow to determine the proper position relationship of as many contigs as possible, in another example of the present invention, the construction system of scaffold further comprises a linearization unit 85. In the linearization unit 85, the scaffold graph is linearized based on the gap size between contigs and the double-barreled relationship between contigs. The step of linearization is as follows: placing each contig at appropriate position in the sub-graph based on the gap size between contigs and the double-barreled relationship between contigs, if no significant overlap exists between any two contigs, then performing linearization according to the position relationship of these two contigs.

The gap size between contigs in the scaffold might be changed due to the linearization of the scaffold graph. In order to present the gap size between contigs in linearized scaffold graph accurately, in another example of the present invention, in the gap size obtaining unit 72, the gap size between contigs in the linearized scaffold graph will be recalculated.

The step of recalculating the gap size between contigs in the linearized scaffold graph comprises: based on the position relationship of the contigs in the linearized scaffold graph, recalculating the gap size between each two adjacent contigs; and relinking adjacent contigs, thereby converting the scaffold graph into a true linear structure. Referring to Figs. 5a and 5b, after converting the linking relationship of AB, EC, AC, ED of Fig. 5a into the linking relationship of AE, EB, BC, CD of Fig. 5b, the gap size of each contigs is calculated from the gap size that has already been obtained. For example, the gap size of AE can be calculated simply as AE=AC-EC.

After performing masking of repeat contigs in the scaffold graph and linearization of sub-graph, it is possible that previously masked repeat contig locates between two unique contigs, since the gap size between contigs in the scaffold have been changed. In this case, in order to reduce the internal gap size in the scaffold and allow the scaffold to be filled as much as possible, the construction system of scaffold further comprises a repeat contig recovering unit 86. In the repeat contig recovering unit 86, when masked repeat contig locates between two unique contigs, the masked repeat contig would be recovered.

Referring to Fig. 6, which shows the scaffold graph obtained in the scaffold construction unit 73. If the previously masked contig R locates between unique contig A and unique contig D of the scaffold graph, the previously masked contig R would be recovered directly.

It is to be noted that, although the repeat contig masking unit 84, the linearization unit 85 and the repeat contig recovering unit 86 are shown simultaneously in Fig. 8, one skilled in the art would understand that, in addition to the double-barreled data mapping unit 71, the gap size obtaining unit 72 and the scaffold construction unit 73, the construction system of fragments assembling scaffold could comprises only the repeat contig masking unit 84 or the linearization unit 85; or both the repeat contig masking unit 84 and the linearization unit 85; or comprises simultaneously the repeat contig masking unit 84, the linearization unit 85 and the repeat contig recovering unit 86.

For better understanding, the above description only shows the relevant part of the examples of the present invention. One skilled in the art would understand that, the construction system of scaffold may be a software unit, hardware unit or a soft-hardware unit that is within a genome sequencing device; or otherwise, integrated as independent configuration into a genome sequencing device or an application system of a genome sequencing device.

In the embodiments of the present invention, by mapping the double-barreled data obtained through sequencing to contigs and obtaining the gap size between said contigs based on multiple pairs of double-barreled data mapped to said contigs, the estimation precision of gap size between contigs in the construction of fragments assembling scaffold is improved greatly. Then the fragments assembling scaffold is constructed based on the gap size between contigs and the double-barreled relation between contigs, and a complete fragments assembling scaffold graph is obtained. As such, even if a genome sequencing method with short sequencing reads is used, it is also possible to finish the task of assembling sequencing fragments. Meanwhile, the error rate in assembling sequencing fragments is reduced. Meanwhile, by masking the repeat contigs in the constructed scaffold graph, mis-assembling due to repeat contigs is avoided, and therefore the accuracy of scaffold construction is greatly improved. By linearization of the constructed scaffold graph, the position relationship of contigs are determined, therefore the coverage length of scaffold is increased. By recovering masked repeat contigs, the information of repeat contigs are used sufficiently, and as many as internal gaps in the scaffold are filled up.

## Claims

1. A computer-implemented method of constructing a scaffold, the method comprising the steps of:
mapping multiple pairs of double-barreled data obtained through sequencing to contigs;
obtaining gap size between said contigs by calculating mean or median length between contigs based on the multiple pairs of double-barreled data mapped to contigs;
constructing a scaffold based on the gap size between contigs and the relative orientation of contigs;
detecting repeat contigs in said scaffold , and masking the detected repeat contigs, wherein repeat contig is a contig that is linked in one direction to a plurality of contigs that have overlaps;
linearizing the scaffold based on the gap size between contigs and the relative orientation of contigs in the scaffold; and
recalculating the gap size between contigs in the linearized scaffold, wherein the recalculation step comprises: based on the position relationship of the contigs in the linearized scaffold, recalculating the gap size between each two adjacent contigs; and relinking adjacent contigs.

2. A system comprising means adapted to perform the method of claim 1.

## Patentansprüche

1. Computerimplementiertes Verfahren zum Konstruieren eines Gerüstes, wobei das Verfahren die folgenden Schritte umfasst:
Abbilden mehrerer Paare doppelläufiger Daten, die durch Sequenzieren erhalten wurden, auf Contigs;
Erhalten der Spaltgröße zwischen den Contigs durch Berechnen des Mittelwertes oder des Medians der Länge zwischen Contigs auf der Grundlage der mehreren Paare doppelläufiger Daten, die auf Contigs abgebildet wurden;
Konstruieren eines Gerüstes auf der Grundlage der Spaltgröße zwischen Contigs und der relativen Orientierung von Contigs;
Detektieren wiederholter Contigs im Gerüst und Maskieren der detektierten wiederholten Contigs, wobei ein wiederholtes Contig ein Contig ist, das in einer Richtung mit mehreren Contigs, die Überlappungen besitzen, verbunden ist;
Linearisieren des Gerüstes auf der Grundlage der Spaltgröße zwischen Contigs und der relativen Orientierung von Contigs im Gerüst; und
Neuberechnen der Spaltgröße zwischen Contigs im linearisierten Gerüst, wobei der Neuberechnungsschritt Folgendes umfasst: Neuberechnen auf der Grundlage der Positionsbeziehungen der Contigs im linearisierten Gerüst der Spaltgröße zwischen jeweils zwei benachbarten Contigs; und
Erneutes Verbinden benachbarter Contigs.

2. System, das Mittel umfasst, die ausgelegt sind, das Verfahren nach Anspruch 1 durchzuführen.

## Revendications

1. Procédé mis en oeuvre par ordinateur pour construire un échafaudage, le procédé comprenant les étapes consistant à :
mettre en correspondance de multiples paires de données à double entrée obtenues par séquençage avec des contigs ;
obtenir une taille de brèche entre lesdits contigs en calculant une longueur moyenne ou médiane entre les contigs en se basant sur les multiples paires de données à double entrée mises en correspondance avec les contigs ;
construire un échafaudage en se basant sur la taille de brèche entre les contigs et l'orientation relative des contigs ;
détecter des contigs répétés dans ledit échafaudage, et masquer les contigs répétés détectés, où un contig répété est un contig qui est lié dans une direction à une pluralité de contigs qui présentent des chevauchements ;
linéariser l'échafaudage en se basant sur la taille de brèche entre les contigs et l'orientation relative des contigs dans l'échafaudage ; et
recalculer la taille de brèche entre les contigs dans l'échafaudage linéarisé, où l'étape de recalcul consiste à : en se basant sur la relation de position des contigs dans l'échafaudage linéarisé, recalculer la taille de brèche entre chaque paire de contigs adjacents ; et lier à nouveau les contigs adjacents.

2. Système comprenant des moyens adaptés pour exécuter le procédé de la revendication 1.
